# EUROPÄISCHE PATENTSCHRIFT

(11) **EP 4 203 850 B1**
(45) Veröffentlichungstag und Bekanntmachung des Hinweises auf die Patenterteilung: **24.09.2025**
(21) Anmeldenummer: 22718602.0
(22) Anmeldetag: 24.03.2022
(51) Int. Cl.: A61F 2/18

(54) **GEHÖRKNÖCHELCHENPROTHESE**
OSSICULAR PROSTHESIS
PROTHÈSE D'OSSELETS

(30) Priorität: 30.03.2021 DE 102021107955
(43) Veröffentlichungstag der Anmeldung: 05.07.2023
(73) Patentinhaber: MED-EL Elektromedizinische Geräte GmbH, 6020 Innsbruck (AT)
(72) Erfinder: STEINHARDT, Uwe, 72145 Hirrlingen (DE); KUEN, Clemens, 6460 Imst (AT); RAINER, Stefan, 6095 Grinzens (AT); MAIR, Lukas, 6130 Schwaz (AT); HAUSCH, Jörg, 72131 Ofterdingen (DE)
(74) Vertreter: Witte, Weller & Partner Patentanwälte mbB
(86) Internationale Anmeldenummer: PCT/EP2022/057793
(87) Internationale Veröffentlichungsnummer: WO 2022/207456

(56) Entgegenhaltungen:
- EP-A1- 1 181 907
- CN-U- 210 228 403
- US-A1- 2007 083 263
- US-B1- 6 398 717

## Beschreibung

Die Erfindung betrifft eine Gehörknöchelchenprothese mit den Merkmalen des Oberbegriffs des Anspruchs 1.

Gehörknöchelchenprothesen dienen als Ersatz eines oder mehrerer Gehörknöchelchen im menschlichen Mittelohr und übertragen anstatt des oder der ersetzten Gehörknöchelchen durch Schallwellen erzeugte Schwingungen vom Trommelfell auf das ovale Fenster des Innenohrs.

Die Patentanmeldung US 2007/0 083 263 A1 offenbart eine Gehörknöchelchenprothese mit einer Kopfplatte, die innen an einem Trommelfell angeordnet wird, einem Fußelement, das an einem Gehörknöchelchen oder einem Innenohr befestigt wird, einem Schaft, der die Kopfplatte und das Fußelement verbindet, und einem Kugelgelenk, das die Kopfplatte gelenkig mit dem Schaft verbindet. Dabei ist eine Kugelpfanne des Kugelgelenks der bekannten Gehörknöchelchenprothese aus einem elastischen Kunststoff gefertigt.

Das Gebrauchsmuster CN 210 228 403 U offenbart eine Gehörknöchelchenprothese mit einer Kopfplatte, von deren einer Seite ein kurzes Zylinderstück axial absteht, das eine Quernut in einer Axialebene aufweist, die von einem radialen Querloch quer durchsetzt wird. In der Quernut ist ein Kugelkopf angeordnet und in einander zugewandte Mündungen des Querlochs eingeschnappt, dessen Durchmesser größer als die Quernut breit ist. Von dem Kugelkopf steht ein Schaft ab, der eine Federklammer an seinem dem Kugelkopf fernen Ende zu einem Aufschnappen auf einen Steigbügel aufweist.

Aufgabe der Erfindung ist eine Gehörknöchelchenprothese mit einem Kugelgelenk mit einer guten Übertragung mechanischer Schwingungen von einem Trommelfell auf ein Gehörknöchelchen eines menschlichen Mittelohrs oder auf ein ovales Fenster eines menschlichen Innenohrs vorzuschlagen. Eine weitere Aufgabe der Erfindung besteht darin, post-operativen Bewegungen, bedingt durch Narbengewebe und dies wiederum hervorgerufen durch den stattfindenden Heilungsprozess am rekonstruierten Trommelfell, zu folgen und damit Druckspitzen, die die akusto-mechanische Signalübertragung negativ beeinträchtigen könnten, zu vermeiden.

Die erfindungsgemäße Gehörknöchelchenprothese mit den Merkmalen des Anspruchs 1 weist ein Kopfelement, ein Fußelement, ein Kugelgelenk, das das Kopfelement und das Fußelement gelenkig verbindet, und einen Schaft auf, der das Kopfelement mit dem Fußelement verbindet, wobei das Kugelgelenk am Kopfelement, am Fußelement oder an an sich beliebiger Stelle des Schafts angeordnet sein kann und vorzugsweise am Kopfelement angeordnet ist.

Das Kopfelement ist ein Befestigungselement, das derart an einer Innenseite eines Trommelfells in einem menschlichen Mittelohr angeordnet wird, dass es durch Schall verursachte mechanische Schwingungen des Trommelfells auf die Gehörknöchelchenprothese überträgt, aber Makrobewegungen, bedingt durch Bewegungen am Trommelfell, wiederum zulässt. Als Trommelfell ist in diesem Zusammenhang sowohl ein vorhandenes, teilweise vorhandenes als auch ein künstlich vollständiges oder teilweise rekonstruiertes Trommelfell sowie ein teilweise vorhandenes und der restliche Teil rekonstruiertes Trommelfell zu verstehen. Die Rekonstruktion umfasst jegliche Form der Herstellung einer schwingfähigen Membran, beispielsweise mit Körpergewebe oder einer künstlich angelegten Schwingungsmembran zur mechanischen Schallübertragung. Mitumfasst ist eine Anordnung oder Befestigung des Kopfelements an einem Gehörknöchelchen oder auch an einem Schwingungserzeuger, der im Ohr schallerzeugende Schwingungen anstelle des Trommelfells erzeugt, anstatt am Trommelfell.

Das Fußelement ist ein Befestigungselement, das an einem Gehörknöchelchen, insbesondere an einem Steigbügel in dem menschlichen Mittelohr oder am ovalen Fenster eines menschlichen Innenohrs angeordnet wird derart, dass es mechanische Schwingungen von der Gehörknöchelchenprothese auf das Gehörknöchelchen oder das ovale Fenster überträgt. Mitumfasst ist eine Anordnung oder Befestigung des Fußelements an einem anderen Gehörknöchelchen als dem Steigbügel oder an anderer Stelle in einem menschlichen Ohr.

Das Kugelgelenk weist eine Kugelaufnahme und einen Kugelkopf auf, der zweidimensional um einen Mittelpunkt des Kugelkopfs schwenkbar in der Kugelaufnahme angeordnet ist. Der Kugelkopf weist den von ihm abstehenden Schaft auf, der allgemein ein Element zu einer Verbindung des Kugelkopfs mit dem Fußelement oder dem Kopfelement ist. Das Kugelgelenk passt eine Winkellage des Kopfelements an eine Lage des Trommelfells in Bezug zu der Gehörknöchelchenprothese an. Zu einer spielfreien Aufnahme des Kugelkopfs in der Kugelaufnahme sieht die Erfindung vor, dass die Kugelaufnahme den Kugelkopf mit einer Vorspannung sowohl in einer radialen als auch in einer axialen Richtung des Schafts in einer Gebrauchsstellung des Schafts hält. Die Gebrauchsstellung ist eine Stellung, die der Schaft in Bezug zu der Kugelaufnahme einnimmt, wenn die Gehörknöchelchenprothese in ein menschliches Ohr implantiert ist. Anstelle der Gebrauchsstellung kann auch eine Stellung des Schafts senkrecht zum Trommelfell oder eine mittlere Schwenkstellung des Schafts gewählt werden. Eine andere Definition des an sich gleichen Sachverhalts ist eine spielfreie Aufnahme des Kugelkopfs in der Kugelaufnahme mit einer Vorspannung sowohl parallel zu einer Trommelfellebene als auch senkrecht zur Trommelfellebene. Die Trommelfellebene ist eine gedachte Tangentialebene des Trommelfells an einer Stelle, an der das Kopfelement der Gehörknöchelchenprothese angeordnet ist. Dabei ist von einer vorgesehenen Anordnung des Kopfelements am Trommelfell auszugehen. Sofern der Schaft des Kugelkopfs senkrecht zur Trommelfellebene ausgerichtet ist, sind beide Definitionen gleichbedeutend. Sofern der Schafts einen Winkel zu einer Senkrechten der Trommelfellebene aufweist, unterscheiden sich die beiden Definitionen um diesen Winkel, der als Toleranz für die Richtungen der Vorspannkräfte aufgefasst werden kann.

Die durch Schall erzeugten mechanischen Schwingungen des Trommelfells werden in einer Längsrichtung des Schafts übertragen. Für eine gute Übertragung der Schwingungen von der Kugelaufnahme auf den Kugelkopf des Kugelgelenks der erfindungsgemäßen Gehörknöchelchenprothese ist deswegen eine möglichst spielfreie unelastische Anordnung des Kugelkopfs in der Kugelaufnahme in der Längsrichtung, also in axialer Richtung des Schafts notwendig, die durch die Vorspannung des Kugelkopfs senkrecht zu der Trommelfellebene oder in der axialen Richtung des Schafts in der Kugelaufnahme erreicht wird. Die mechanischen Schwingungen werden dadurch nicht oder allenfalls wenig gedämpft.

Eine Ausgestaltung der Erfindung sieht ein elastisches Vorspannelement auf einer in Bezug auf den Kugelkopf dem Schaft gegenüberliegenden Seite vor, das den Kugelkopf in Richtung des Schafts beaufschlagt. Diese Ausgestaltung der Erfindung erziehlt eine definierte Lage des Kugelkopfs in der Kugelaufnahme und bewirkt eine gute Schwingungsübertragung in einer Schwingungsrichtung.

Eine einfache Ausführung, die eine kompakte Kugelaufnahme, ein kompaktes Kugelgelenk und ein kompaktes Kopfelement ermöglicht, sieht eine Federscheibe als Vorspannelement vor, die senkrecht zu einer von ihr definierten Fläche elastisch und die tangential zum Kugelkopf angeordnet ist. Die Federscheibe kann kreisrund, rund, eckig, streifenförmig und an sich eine beliebige Form aufweisen, sie kann lochfrei sein oder ein Mittelloch oder ein oder mehrere andere Löcher aufweisen. Möglich ist beispielsweise auch eine Federscheibe mit einem Schlitz und vorzugsweise mehreren sternförmig angeordneten Schlitzen. Durch ihre Flächenform lässt sich die Federscheibe platzsparend tangential zum Kugelkopf an oder in der Kugelaufnahme anordnen. Durch Durchbrüche wie dem Loch oder Mittelloch oder einen oder mehrere Schlitze lassen sich Federeigenschaften der Federscheibe beeinflussen, insbesondere ihre Federhärte ändern. Ein Mittelloch in der Federscheibe oder sternförmig, insbesondere radial angeordnete Schlitze oder allgemein Schlitze, die sich in einer Mitte der Federscheibe treffen, zentrieren den Kugelkopf des Kugelgelenks in der Kugelaufnahme der Gehörknöchelchenprothese.

Eine Ausgestaltung der Erfindung sieht eine Ausbildung der Kugelaufnahme als Schnappeinrichtung vor, in die der Kugelkopf vergleichbar einem Druckknopf eingeschnappt ist. Die Kugelaufnahme weist bei einer Ausgestaltung der Erfindung Schnappelemente vor, die über einen Umfang verteilt um den Kugelkopf herum angeordnet sind und zwischen die der Kugelkopf eingeschnappt ist. Die Schnappelemente können in Umfangsrichtung gleichmäßig oder ungleichmäßig verteilt angeordnet sein und/oder sie können beispielsweise fingerförmig oder streifenförmig und radial zum Kugelkopf federnd sein. Die Schnappelemente liegen mit einer parallel zu dem Trommelfell oder radial zu dem Schaft in der Gebrauchsstellung wirkenden Vorspannung an dem Kugelkopf an und bewirken den parallel zu dem Trommelfell oder radial zu dem Schaft in der Gebrauchsstellung spielfreien Halt des Kugelkopfs in der Kugelaufnahme. Ein weiterer Vorteil der Ausbildung der Kugelaufnahme als Schnappeinrichtung ist ein einfacher Zusammenbau des Kugelgelenks.

In bevorzugter Ausgestaltung der Erfindung bestehen die Kugelaufnahme und der Kugelkopf aus Metall oder Keramik und liegen unmittelbar ohne Zwischenlage eines anderen Elements, das die Schwingungsübertragung von der Kugelaufnahme auf den Kugelkopf oder umgekehrt dämpfen oder in anderer Weise beeinträchtigen könnte, aneinander an. Diese Ausgestaltung der Erfindung bewirkt eine gute Schwingungsübertragung mit geringen Verlusten. Der Kugelkopf und die Kugelaufnahme können beispielsweise aus Titanlegierungen oder Edelstahl bestehen.

Sämtliche in der Beschreibung genannte und/oder der Zeichnung dargestellte Merkmale können einzeln für sich oder in jeder beliebigen Kombination bei Ausführungen der Erfindung verwirklicht sein. Ausführungen der Erfindung, die nicht alle, sondern nur einen Teil der Merkmale eines Anspruchs, auch des unabhängigen Anspruchs, aufweisen, sind möglich.

Die Erfindung wird nachfolgend anhand eines in der Zeichnung dargestellten Ausführungsbeispiels näher erläutert. Es zeigen:
- Figur 1: eine Gehörknöchelchenprothese gemäß der Erfindung in einer perspektivischen Explosionsdarstellung;
- Figur 2: die Gehörknöchelchenprothese aus Figur 1 in zusammengesetztem Zustand in einer perspektivischen Darstellung;
- Figur 3: die Gehörknöchelchenprothese aus Figur 2 mit einer anderen Blickrichtung;
- Figur 4: einen Achsschnitt eines Kopfelements und eines Kugelkopfs der Gehörknöchelchenprothese aus Figuren 1 und 2 in größerer Darstellung;
- Figuren 5 und 6: Abwandlungen der Gehörknöchelchenprothese aus Figuren 1 bis 4 gemäß der Erfindung in Figur 2 entsprechenden perspektivischen Darstellungen.

Grundsätzlich sind in den Figuren gleiche Teile mit gleichen Bezugszahlen versehen.

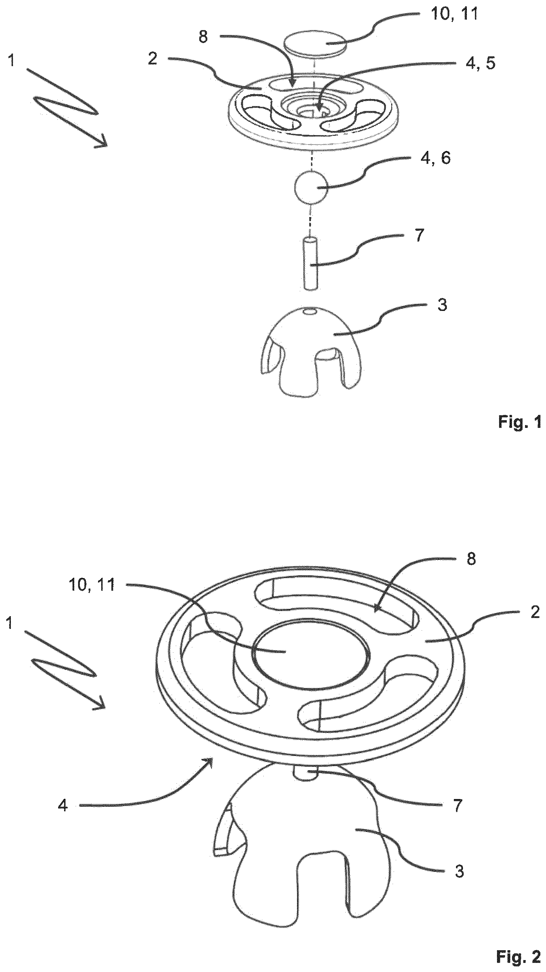

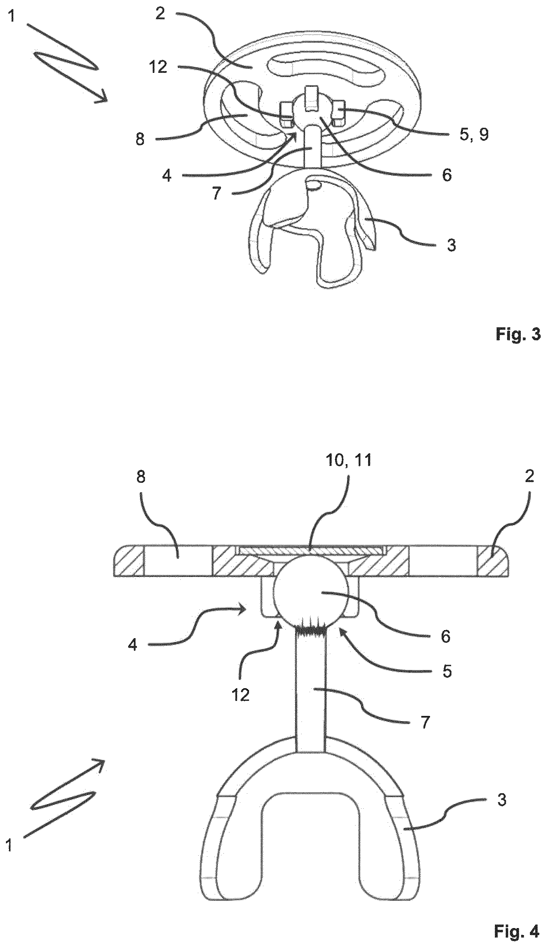

Die in Figuren 1 bis 4 dargestellte, erfindungsgemäße Gehörknöchelchenprothese 1 ist zum Ersatz eines oder mehrerer Gehörknöchelchen in einem menschlichen Ohr vorgesehen. Sei weist ein Kopfelement 2, das zu einer Anordnung an einer Innenseite eines Trommelfells des Ohrs, und ein Fußelement 3, das zu einer Anbringung an einem Gehörknöchelchen, insbesondere an einem Steigbügel oder einem ovalen Fenster im Mittelohr beziehungsweise Innenohr eines Menschen vorgesehen ist, auf. Die Gehörknöchelchenprothese 1 überträgt durch Schallwellen erzeugte Schwingungen des Trommelfells auf den Steigbügel oder das Element des Ohrs, an dem das Fußelement 3 angebracht ist. Als Trommelfell ist in diesem Zusammenhang sowohl ein vorhandenes, teilweise vorhandenes als auch ein künstlich vollständiges oder teilweise rekonstruiertes Trommelfell sowie ein teilweise vorhandenes und der restliche Teil rekonstruiert zu verstehen. Die Rekonstruktion umfasst jegliche Form der Herstellung einer schwingfähigen Membran, beispielsweise mit Körpergewebe oder einer künstlich angelegten Schwingungsmembran zur mechanischen Schallübertragung. Das Kopfelement kann auch zu einer Anbringung an einem Schwingungserzeuger ausgebildet sein, der Schwingungen erzeugt, die im Ohr Schall erzeugen (nicht dargestellt).

Die Gehörknöchelchenprothese 1 weist das Kopfelement 2, das Fußelement 3, ein Kugelgelenk 4 mit einer Kugelaufnahme 5, einem Kugelkopf 6 und einem Schaft 7 auf. Im Ausführungsbeispiel ist die Kugelaufnahme 5 Bestandteil des Kopfelements 2. Der Schaft 7 steht radial von dem Kugelkopf 6 ab, er ist im Ausführungsbeispiel mit dem Kugelkopf 6 verschweißt, kann aber auch in anderer Weise, beispielsweise durch Einschrauben starr mit dem Kugelkopf 6 verbunden sein. Das Kugelgelenk 4 verbindet den Schaft 7 zweidimensional um einen Mittelpunkt des Kugelkopfs 6 schwenkbar mit dem Kopfelement 2. Der Schaft 7 verbindet das Fußelement 3 starr mit dem Kugelkopf 6. Dadurch sind das Kopfelement 2 und das Fußelement 3 zweidimensional um den Mittelpunkt des Kugelkopfs 6 schwenkbar und in festem Abstand voneinander miteinander verbunden.

Das Kopfelement 2 der Gehörknöchelchenprothese 1 ist im dargestellten und beschriebenen Ausführungsbeispiel der Erfindung eine kreisförmige, ebene Scheibe mit einem Mittelloch und kreisbogenförmig in Umfangsrichtung verlaufenden Langlöchern 8. Andere Ausführungen des Kopfelements 2 sind möglich, wobei auch Ausführungen, die zu einer Anordnung an einem Schwingungserzeuger anstatt an der Innenseite des Trommelfells ausgebildet sind, möglich sind (nicht dargestellt).

Das Fußelement 3 der Gehörknöchelchenprothese 1 ist im Ausführungsbeispiel glockenförmig, wobei andere Ausführungen möglich sind.

Der Schaft 7 ist im Ausführungsbeispiel ein Zylinderstift, an dessen dem Kugelkopf 6 fernen Ende das Fußelement 3 angeschweißt oder in anderer Weise starr befestigt ist.

Das Kopfelement 2 der Gehörknöchelchenprothese 1 weist die Kugelaufnahme 5 auf, in der der Kugelkopf 6 zweidimensional um seinen Mittelpunkt schwenkbar einliegt. Die Kugelaufnahme 5 und der Kugelkopf 6 bilden das Kugelgelenk 4.

Im Ausführungsbeispiel ist die Kugelaufnahme 5 als Schnappeinrichtung ausgebildet, in die der Kugelkopf 6 vergleichbar einem Druckknopf eingeschnappt ist. Im Ausführungsbeispiel weist die Kugelaufnahme 5 vier fingerförmige Schnappelemente 9 auf, die um das Mittelloch des Kopfelements 2 herum angeordnet sind und von einer Seite des Kopfelements 2 abstehen. Ein Abstand der Schnappelemente 9 voneinander ist kleiner als ein Durchmesser des Kugelkopfs 6, so dass die Schnappelemente 9 mit einer elastischen Federkraft am Kugelkopf 6 anliegen, die in Richtung des Mittelpunkts des Kugelkopfs 6 gerichtet ist. Die elastische Federkraft der Schnappelemente 9 kann auch als Vorspannung parallel zum Kopfelement 2 aufgefasst werden. Dabei definiert das im Ausführungsbeispiel scheibenförmige Kopfelement 2 eine Trommelfellebene, die eine Tangentialebene eines Trommelfells an einer Stelle ist, an der das Kopfelement 2 am Trommelfell angeordnet ist, wenn das Kopfelement 2 in vorgesehener Weise an dem Trommelfell angeordnet ist. Die Vorspannung der Schnappelemente 9 wirkt auch radial zu dem Schaft 7, wenn dieser senkrecht zum Kopfelement 2 ausgerichtet ist, was als Gebrauchsstellung des Schafts 7 angesehen werden kann. Die Gebrauchsstellung des Schafts 7 ist die Stellung, die der Schaft 7 einnimmt, wenn die Gehörknöchelchenprothese 1 in ein Ohr implantiert ist.

Dem Kopfelement 2 ferne Enden der Schnappelemente 9 sind etwas hakenförmig nach innen in Richtung des Kugelkopfs 6 stehend ausgebildet, so dass sie den Kugelkopf 6 auf einer dem Kopfelement 2 abgewandten Seite untergreifen und in der Kugelaufnahme 5 halten.

Die Anzahl von vier Schnappelementen 9 ist nicht zwingend für die Erfindung, es können auch weniger oder mehr Schnappelemente 9 vorgesehen sein und die Schnappelemente 9 können in Umfangsrichtung des Kugelkopfs 6 auch ungleichmäßig anstatt gleichmäßig verteilt angeordnet sein (nicht dargestellt).

Im Mittelloch des Kopfelements 2 ist eine senkrecht zu ihrer Fläche elastische Federscheibe 10 als Vorspannelement 11 angeordnet, die im Ausführungsbeispiel durch Schweißen mit dem Kopfelement 2 verbunden ist, allerdings auch in anderer Weise am Kopfelement 2 beziehungsweise der Kugelaufnahme 5 festgelegt sein kann. Das Vorspannelement 11 ist auf einer dem Schaft 7 abgewandten Seite des Kugelkopfs 6 tangential zum Kugelkopf 6 angeordnet, es verschließt die Kugelaufnahme 5 auf einer dem Schaft 7 gegenüberliegenden Seite des Kugelkopfs 6. Ein Abstand der das Vorspannelement 11 bildenden Federscheibe 10 von den hakenförmigen Enden 12 der Schnappelemente 9, die den Kugelkopf 6 auf der dem Vorspannelement 11 abgewandten Seite untergreifen, ist so klein gewählt, dass der Kugelkopf 6 das Vorspannelement 11 elastisch verformt, wodurch das Vorspannelement 11 eine elastische Kraft senkrecht zum Kopfelement 2 und in Längsrichtung des Schafts 7 auf den Kugelkopf 6 ausübt. Diese elastische Kraft kann als Vorspannung senkrecht zum Kopfelement 2 beziehungsweise senkrecht zu der Trommelfellebene und in Längsrichtung des Schafts 7 aufgefasst werden. Die Vorspannung, die das Vorspannelement 11 auf den Kugelkopf 6 ausübt, hält den Kugelkopf 6 spielfrei senkrecht zum Kopfelement 2 und in der Längsrichtung des Schafts 7, also in der Richtung, in der Schwingungen des Trommelfells über die Gehörknöchelchenprothese 1 auf den Steigbügel, ein anderes Gehörknöchelchen oder das ovale Fenster übertragen werden. Diese Spielfreiheit des Kugelgelenks 4 der erfindungsgemäßen Gehörknöchelchenprothese 1 bewirkt eine nahezu verlustfreie Schwingungsübertragung vom Kopfelement 2 zum Fußelement 3 oder jedenfalls im Kugelgelenk 4 der Gehörknöchelchenprothese 1.

Die Bauteile der Gehörknöchelchenprothese 1, insbesondere die Kugelaufnahme 5 und der Kugelkopf 6 bestehen aus Metall oder Keramik, beispielsweise aus Edelstahl oder einer Titanlegierung. Der Kugelkopf 6 liegt unmittelbar und ohne irgendein Zwischenelement, das die Schwingungsübertragung dämpfen oder in anderer Weise verschlechtern könnte, in der Kugelaufnahme 5 beziehungsweise zwischen den Schnappelementen 9 und der das Vorspannelement 11 bildenden Federscheibe 10 ein, wodurch eine gute und weitgehend verlustfreie Schwingungsübertragung vom Kopfelement 2 auf den Kugelkopf 6 erreicht wird.

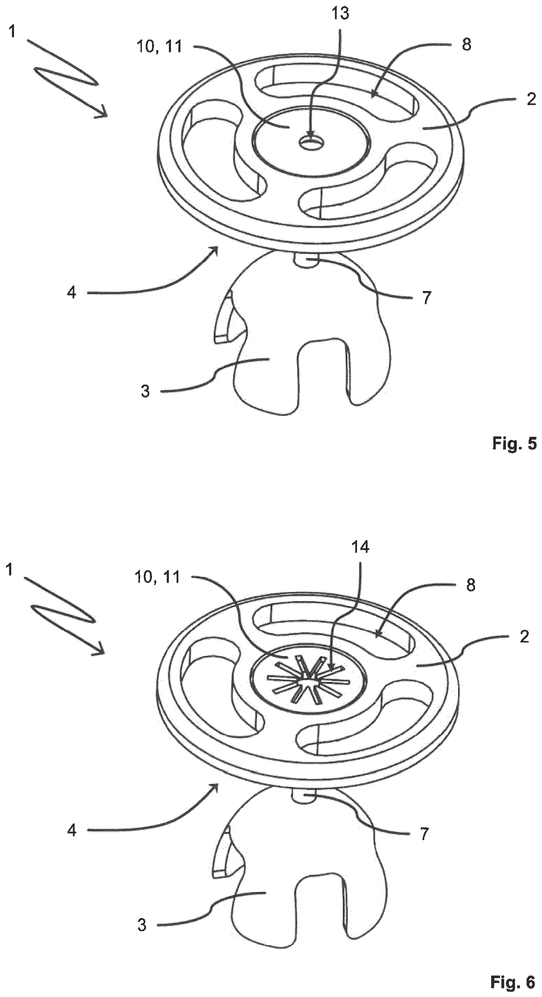

Bei der in Figur 5 dargestellten Ausführungsform einer erfindungsgemäßen Gehörknöchelchenprothese 1 weist die das Vorspannelement 11 bildende Federscheibe 10 ein Mittelloch 13 auf, das die Federscheibe 10 beim Schweißen an dem Kugelkopf 6 zentriert.

Bei der in Figur 6 dargestellten Ausführungsform einer erfindungsgemäßen Gehörknöchelchenprothese 1 weist die das Vorspannelement 11 bildende Federscheibe 10 radiale Schlitze 14 auf, die sich in einer Mitte der Federscheibe 10 treffen und das Federelement beim Schweißen an dem Kugelkopf 6 zentrieren.

Das Mittelloch 13 und die Schlitze 14 beeinflußen durch ihre Form, Größe und Anordnung in der Federscheibe 10 deren Federeigenschaften, insbesondere eine Federhärte der Federscheibe 10.

## Patentansprüche

1. Gehörknöchelchenprothese, mit einem Kopfelement (2), das zu einer Anordnung an einem Trommelfell vorgesehen ist, mit einem Fußelement (3), das zu einer Anordnung an einem Gehörknöchelchen im menschlichen Mittelohr oder an dem ovalen Fenster eines menschlichen Innenohrs vorgesehen ist, und mit einem Kugelgelenk (4), das das Kopfelement (2) und das Fußelement (3) gelenkig verbindet, wobei das Kugelgelenk (4) eine Kugelaufnahme (5) und einen Kugelkopf (6) aufweist, der zweidimensional um seinen Mittelpunkt schwenkbar in der Kugelaufnahme (5) einliegt, wobei der Kugelkopf (6) einen von ihm abstehenden Schaft (7) aufweist und die Kugelaufnahme (5) den Kugelkopf (6) mit einer Vorspannung parallel zu einer Trommelfellebene oder radial zu dem Schaft (7) in einer Gebrauchsstellung des Schafts (7) hält und wobei die Kugelaufnahme (5) den Kugelkopf (6) spielfrei mit einer Vorspannung senkrecht zu der Trommelfellebene oder in einer Längsrichtung des Schafts (7) in der Gebrauchsstellung des Schafts (7) hält, die Kugelaufnahme (5) ein elastisches Vorspannelement (11) auf einer dem Schaft (7) gegenüberliegenden Seite des Kugelkopfs (6) aufweist, das den Kugelkopf (6) in Richtung des Schafts (7) beaufschlagt, und **dadurch gekennzeichnet, dass** das Vorspannelement (11) eine tangential zum Kugelkopf (6) angeordnete Federscheibe (10) ist.

2. Gehörknöchelchenprothese nach Anspruch 1, **dadurch gekennzeichnet, dass** die Federscheibe (10) ein Loch, insbesondere ein Mittelloch (13) oder einen Schlitz, insbesondere sternförmig angeordnete Schlitze (14) aufweist.

3. Gehörköchelchenprothese nach Anspruche 1 oder 2, **dadurch gekennzeichnet, dass** die Kugelaufnahme (5) Schnappelemente (9) aufweist, die über einen Umfang verteilt angeordnet sind, zwischen die der Kugelkopf (6) eingeschnappt ist und die mit der Vorspannung parallel zu der Trommelfellebene oder radial zu dem Schaft (7) in der Gebrauchsstellung des Schafts (7) an dem Kugelkopf (6) anliegen.

4. Gehörknöchelchenprothese nach einem oder mehreren der vorhergehenden Ansprüche, **dadurch gekennzeichnet, dass** die Kugelaufnahme (5) und der Kugelkopf (6) aus Metall oder Keramik bestehen und unmittelbar aneinander anliegen.

## Claims

1. Auditory ossicle prosthesis, comprising a head element (2), which is intended for arrangement on an eardrum, a foot element (3), which is intended for arrangement on an auditory ossicle in the human middle ear or on the oval window of a human inner ear, and a ball joint (4) which connects the head element (2) and the foot element (3) in an articulated manner, the ball joint (4) comprises a ball receptacle (5) and a ball head (6) which is pivotable two-dimensionally about its center within the ball receptacle (5), wherein the ball head (6) comprises a shaft (7) projecting from it and the ball receptacle (5) holds the ball head (6) with a pre-load parallel to a plane of the eardrum or radially to the shaft (7) in a use position of the shaft (7) and the ball receptacle (5) holds the ball head (6) without play with a pre-load perpendicular to the plane of the eardrum or in a longitudinal direction of the shaft (7) in a use position of the shaft (7), the ball receptacle (5) comprises an elastic pre-loading element (11) on a side of the ball head (6) opposite the shaft (7) which acts on the ball head (6) in the direction of the shaft (7), **characterized in that** and the pre-loading element (11) is a spring washer (10) arranged tangentially to the ball head (6).

2. Auditory ossicle prosthesis according to claim 1, **characterized in that** the spring washer (10) comprises a hole, in particular a central hole (13) or a slot, in particular slots (14) arranged in a star shape.

3. Auditory ossicle prosthesis according to claim 1 or 2, **characterized in that** the ball receptacle (5) comprises snap elements (9) which are arranged distributed over a circumference, between which the ball head (6) is snapped in and which rest on the ball head (6) with the pre-load parallel to the plane of the eardrum or radially to the shaft (7) in the use position of the shaft (7).

4. Auditory ossicle prosthesis according to one or more of the preceding claims, **characterized in that** the ball receptacle (5) and the ball head (6) are made of metal or ceramic and lie directly against one another.

## Revendications

1. Prothèse pour osselet, comportant un élément formant tête (2) prévu pour une disposition sur un tympan, comportant un élément formant pied (3) prévu pour une disposition sur un osselet dans l'oreille moyenne humaine ou sur la fenêtre ovale d'une oreille interne humaine, et comportant une articulation à rotule (4) qui relie de manière articulée l'élément formant tête (2) et l'élément formant pied (3), dans laquelle l'articulation à rotule (4) présente un logement pour rotule (5) et une tête formant rotule (6) qui repose dans le logement pour rotule (5) de manière à pouvoir pivoter en deux dimensions autour de son point central, dans laquelle la tête formant rotule (6) présente une tige (7) faisant saillie depuis celle-ci et le logement pour rotule (5) maintient la tête formant rotule (6) avec une précontrainte parallèlement à un plan de tympan ou radialement à la tige (7) dans une position d'utilisation de la tige (7) et dans laquelle le logement pour rotule (5) maintient la tête formant rotule (6) sans jeu avec une précontrainte perpendiculairement au plan de tympan ou dans une direction longitudinale de la tige (7) dans la position d'utilisation de la tige (7), le logement pour rotule (5) présente un élément de précontrainte (11) élastique sur un côté de la tête formant rotule (6) opposé à la tige (7), lequel élément de précontrainte sollicite la tête formant rotule (6) en direction de la tige (7), et **caractérisée en ce que** l'élément de précontrainte (11) est une rondelle élastique (10) disposée tangentiellement à la tête formant rotule (6).

2. Prothèse pour osselet selon la revendication 1, **caractérisée en ce que** la rondelle élastique (10) présente un trou, en particulier un trou central (13) ou une fente, en particulier des fentes (14) disposées en étoile.

3. Prothèse pour osselet selon la revendication 1 ou 2,
**caractérisée en ce que** le logement pour rotule (5) présente des éléments d'encliquetage (9) qui sont disposés de manière à être répartis sur une circonférence, entre lesquels la tête formant rotule (6) est encliquetée, et qui s'appuient contre la tête formant rotule (6) avec la précontrainte parallèlement au plan de tympan ou radialement à la tige (7) dans la position d'utilisation de la tige (7).

4. Prothèse pour osselet selon l'une ou plusieurs des revendications précédentes, **caractérisée en ce que** le logement pour rotule (5) et la tête formant rotule (6) se composent de métal ou de céramique et s'appuient directement l'un contre l'autre.
